# EUROPEAN PATENT SPECIFICATION

(11) **EP 2 730 918 B1**
(45) Date of publication and mention of the grant of the patent: **14.09.2016**
(21) Application number: 12811175.4
(22) Date of filing: 05.07.2012
(51) Int. Cl.: H01J 49/16

(54) **MATRIX FOR MALDI MASS SPECTROMETRY AND MALDI MASS SPECTROMETRY METHOD**
MATRIX FÜR DIE MALDI-MASSENSPEKTROMETRIE UND MALDI-MASSENSPEKTROMETRIEVERFAHREN
MATRICE POUR SPECTROMÉTRIE DE MASSE MALDI ET MÉTHODE DE SPECTROMÉTRIE DE MASSE MALDI

(30) Priority: 08.07.2011 JP 2011152328
(43) Date of publication of application: 14.05.2014
(73) Proprietor: Kyushu University, National University Corporation, Fukuoka-shi, Fukuoka 812-8581 (JP)
(72) Inventor: SHINDO, Mitsuru, Fukuoka-shi Fukuoka 812-8581 (JP); WARIISHI, Hiroyuki, Fukuoka-shi Fukuoka 812-8581 (JP); MIURA, Daisuke, Fukuoka-shi Fukuoka 812-8581 (JP); FUJIMURA, Yoshinori, Fukuoka-shi Fukuoka 812-8581 (JP)
(74) Representative: Winter, Brandl, Fürniss, Hübner, Röss, Kaiser, Polte - Partnerschaft mbB
(86) International application number: PCT/JP2012/067227
(87) International publication number: WO 2013/008723

(56) References cited:
- EP-A1- 2 157 432
- EP-A2- 0 326 328
- EP-A2- 0 326 330
- WO-A1-03/040715
- WO-A1-03/050517
- WO-A2-2006/034235
- DE-A1- 10 238 069
- JP-A- 2009 257 848
- SYLVAIN PELLEGRINI ET AL: "A novel multicomponent reaction: easy access to ferrocenyl (alkylimino)-1,4-dihydroquinolines", TETRAHEDRON LETTERS, PERGAMON, GB, vol. 52, no. 15, 28 January 2011 (2011-01-28), pages 1742-1744, XP028176384, ISSN: 0040-4039, DOI: 10.1016/J.TETLET.2011.01.144 [retrieved on 2011-02-04]
- DE SOUZA M V N ET AL: "Synthesis and in vitro antitubercular activity of a series of quinoline derivatives", BIOORGANIC & MEDICINAL CHEMISTRY, PERGAMON, GB, vol. 17, no. 4, 15 February 2009 (2009-02-15), pages 1474-1480, XP025949552, ISSN: 0968-0896, DOI: 10.1016/J.BMC.2009.01.013 [retrieved on 2009-01-15]
- VERMILLION-SALSBURY, R.L.: '9-Aminoacrydine as a matrix for negative mode matrix-assisted laser desorption/ionization' RAPID COMMUN. MASS SPECTROM. vol. 16, no. 16, 2002, pages 1575 - 1581, XP002569097
- ROBINS, C.: 'The use of nonpolar matrices for matrix-assisted laser desorption/ionization mass spectrometric analysis of high boiling crude oil fractions' RAPID COMMUN. MASS SPECTROM. vol. 17, no. 24, 2003, pages 2839 - 2845, XP055127007
- NONAMI, H.: 'Evaluation of pyridoindoles, pyridylindoles and pyridylpyridoindoles as matrices for ultraviolet matrix-assisted laser desorption/ionization time-of-flight mass spectrometry' RAPID COMMUN. MASS SPECTROM. vol. 15, no. 23, 2001, pages 2354 - 2373, XP009023792
- MACHA, S.F.: 'Application of Nonpolar Matrices for the Analysis of Low Molecular Weight Nonpolar Synthetic Polymers by Matrix-Assisted Laser Desorption/Ionization Time-of-Flight Mass Spectrometry' J AM SOC MASS SPECTROM vol. 11, no. 8, 2000, pages 731 - 737, XP004210139
- MACHA, S.F.: 'Influence of ionization energy on charge-transfer ionization in matrix-assisted laser desorption/ionization mass spectrometry' ANALYTICA CHIMICA ACTA vol. 397, no. 1-3, 1999, pages 235 - 245, XP055127013

## Description

### TECHNICAL

The present invention relates to a matrix used for ionizing a material to be analyzed in matrix-assisted laser desorption/ionization (MALDI) mass spectrometry and to a MALDI mass spectrometry method using the matrix.

### BACKGROUND ART

Matrix-assisted laser desorption/ionization (MALDI) mass spectrometry is soft ionization mass spectrometry that is widely used to analyze a biological molecule rapidly. The use of MALDI mass spectrometry makes it possible to make a highly precise analysis of, for example, a high-molecular-weight protein, which has not easily been attained by any other ionizing method. Accordingly, this mass spectrometry has been used mainly to make mass spectrometry of biological polymers.

In MALDI mass spectrometry, a mixed crystal of a material to be analyzed and a matrix is prepared, and the crystal is irradiated with a laser beam to ionize the material to be analyzed. The matrix absorbs the light energy of the laser to be ionized, and is simultaneously heated rapidly to be gasified. By the irradiation with the laser, molecules of the sample are not directly gasified. However, these molecules are desorbed together with the matrix molecules surrounding the sample molecules. Subsequently, protons, electrons and others are exchanged between the ionized matrix molecules and sample molecules, so that the material to be analyzed is ionized. As a source for the laser, a nitrogen laser (wavelength: 337 nm) or YAG laser (wavelength: 355 nm) is generally used; thus, as the matrix, a substance having an absorption band in this wavelength region is used.

In recent years, MALDI mass spectrometry has been used also to analyze low-molecular-weight compounds. The spectrometry can attain a rapid analysis and a microanalysis, and can further be applied to molecular imaging. For this reason, the spectrometry has been expected to be applied to metabolome analysis. Whether or not a MALDI mass spectrometry succeeds depends largely on the performance of a matrix therefor. Thus, demands for a matrix suitable for the analysis of low-molecular-weight compounds have been increasing. For example, Patent Document 1 suggests a 1H-tetrazole derivative as a matrix suitable for cationizing low-molecular-weight compounds.

Many biological low-molecular-weight molecules are anionic compounds, such as carboxylic acids, amino acids, and phosphates. Thus, the importance of the development of a matrix suitable for a negative ion mode for detecting anions has been increasing. For example, Non-Patent Document 1 discloses that 9-aminoacridine is suitable as a matrix for MALDI mass spectrometry in a negative ion mode.

### PRIOR ART DOCUMENTS

### PATENT DOCUMENT

Patent Document 1: JP 2010-204050 A

### NON-PATENT DOCUMENT

Non-Patent Document 1: "9-Aminoacrydine as a matrix for negative mode matrix-assisted laser desorption/ionization", Rachal L. Vermillion-Salsbury and David M. Hercules, Rapid Communications in Mass Spectrometry, vol. 16, No. 16, pp. 1,575-1,581, published on August 30, 2002 by John Wiley & Sons Co.

### SUMMARY OF THE INVENTION

### PROBLEMS TO BE SOLVED BY THE INVENTION

However, the IH-tetrazole derivative described in Patent Document 1 is a matrix for MALDI mass spectrometry in a positive ion mode. It is unclear whether or not the derivative is applicable to the negative ion mode. 9-Aminoacridine described in Non-Patent Document 1 is currently the most popular as a matrix for MALDI mass spectrometry in a negative ion mode. However, according to the matrix, many compounds are not measurable. Thus, this compound is not necessarily an optimal matrix. As described above, although demands for a matrix suitable for negative-ion-mode MALDI mass spectrometry for low-molecular-weight compounds have been increasing, there has not yet been a matrix having versatility in the present circumstances.

The present invention has been made in light of such problems, and an object thereof is to provide a matrix for MALDI mass spectrometry that has a high ability of ionizing low-molecular-weight compounds, and makes it possible to make measurement in a negative ion mode, and a MALDI mass spectrometry method using the matrix.

### SOLUTIONS TO THE PROBLEMS

The present invention provides a matrix for MALDI mass spectrometry according to any one of the following items [1] to [5].
[1] A matrix for matrix-assisted laser desorption/ionization (MALDI) mass spectrometry, comprising:
   a compound represented by the following 18 to 21, 25, 26, 41 or 42, or the salts thereof: 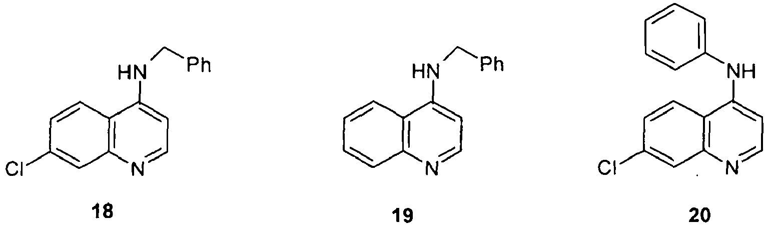 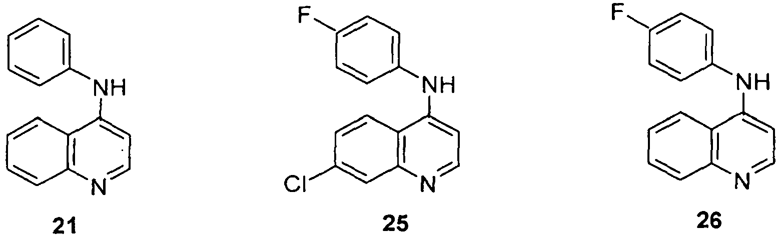 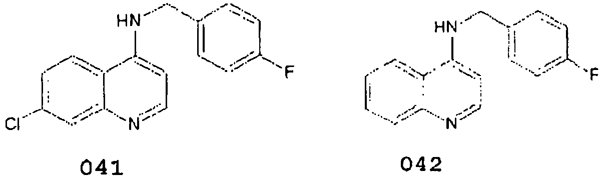
[2] The matrix for matrix-assisted laser desorption/ionization (MALDI) mass spectrometry according to item [1], comprising:
   the compound represented by the following 18, or the salts thereof: 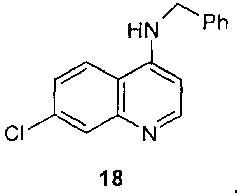
[3] The matrix for matrix-assisted laser desorption/ionization (MALDI) mass spectrometry according to item [1], comprising:
   the compound represented by the following 41 or 42, or the salts thereof: 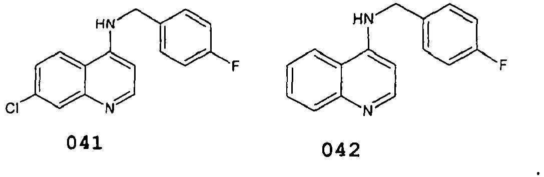
[4] A MALDI mass spectrometry method of making MALDI mass spectrometry in a negative ion mode using the matrix according to any one of items [1], [2] and [3].
[5] The MALDI mass spectrometry method according to item [4], wherein a material to be analyzed is an organic compound having a molecular weight of 1000 or less.

### EFFECTS OF THE INVENTION

According to the present invention, a novel matrix for MALDI mass spectrometry which has a higher ability of ionizing many low-molecular-weight compounds, in particular, biological low-molecular-weight compounds than 9-aminoacridine and further makes it possible to attain mass spectrometry in a negative ion mode with a high sensitivity and a MALDI mass spectrometry method using the matrix are provided. Since the matrix of the invention for MALDI mass spectrometry makes it possible to attain high-sensitivity MALDI mass spectrometry of biological molecules or metabolites thereof, the matrix can be used suitably for analyzing a metabolome, and for others.

### BRIEF DESCRIPTION OF THE DRAWINGS

Fig. 1 is a mass spectrum showing a result of a blank measurement of 9-aminoanthracene (17) which compound is not comprised by the present invention.
Fig. 2 is a mass spectrum showing a result of a blank measurement of 9-aminoanthracene (17) which compound is not comprised by the present invention.
Fig. 3 is a mass spectrum showing a result obtained by using 9-aminoanthracene (17), which compound is not comprised by the present invention, as a matrix to make MALDI mass spectrometry of a mixture (see Table 2) of anionic biological components.
Fig. 4 is a mass spectrum showing a result obtained by using 9-aminoacridine (9-AA) as a matrix to make MALDI mass spectrometry of a mixture (see Table 2) of anionic biological components.
Fig. 5 is a mass spectrum showing a result of a blank measurement of 7-chloro-4-(N-benzylamino)quinoline (18) which compound is comprised by the present invention.
Fig. 6 is a mass spectrum showing a result obtained by using 7-chloro-4-(N-benzylamino)quinoline (18), which compound is comprised by the present invention, as a matrix to make MALDI mass spectrometry of a mixture (see Table 3) of anionic biological components.
Fig. 7 is a mass spectrum showing a result obtained by using 9-aminoacridine (9-AA) as a matrix to make MALDI mass spectrometry of a mixture (see Table 3) of anionic biological components.
Fig. 8 is a mass spectrum showing a result obtained by using 9-aminoacridine (9-AA) as a matrix to make MALDI mass spectrometry of cis-cinnamic acid.
Fig. 9 is a mass spectrum showing a result obtained by using 4-(N-p-fluorobenzyl)amino-7-chloroquinoline (41)), which compound is comprised by the present invention, as a matrix to make MALDI mass spectrometry of cis-cinnamic acid.
Fig. 10 is a mass spectrum showing a result obtained by using 4-(N-p-fluorobenzylamino)quinoline (42)), which compound is comprised by the present invention, as a matrix to make MALDI mass spectrometry of cis-cinnamic acid.

### EMBODIMENTS OF THE INVENTION

A matrix for MALDI mass spectrometry according to an embodiment of the present invention is a compound having a structure represented by the following formula (18), (19), (20), (21), (25), (26), (41) and (42), or their salts thereof:

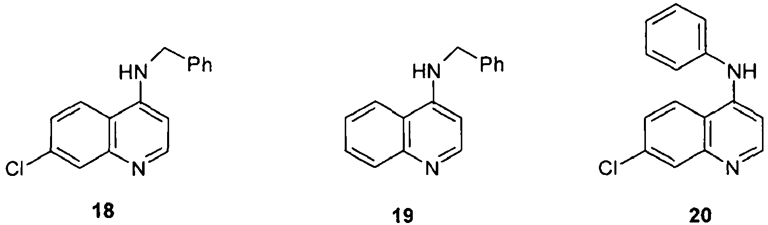

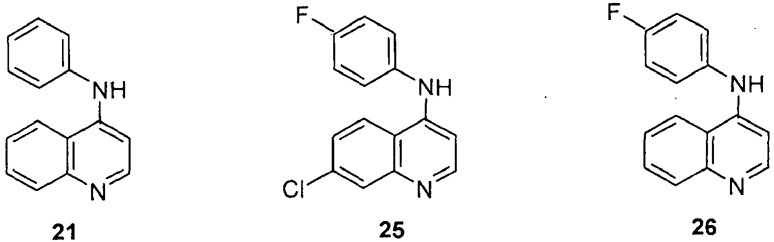

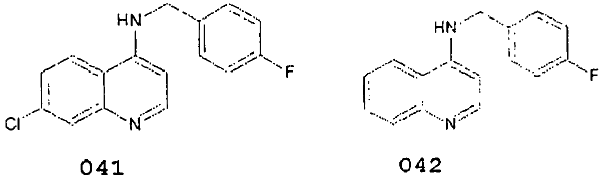

Compounds of the matrix for MALDI mass spectrometry are partially commercially available. Compounds that are not commercially available can be synthesized from the commercially available compounds, respectively, through several steps by any known method.

The thus obtained matrix for MALDI mass spectrometry can be handled in the same way as ordinarily used matrices. For example, a measurement sample for MALDI mass spectrometry can be prepared by dissolving a material to be analyzed and the matrix in any appropriate solvent such as acetonitrile or THF, dropping the resultant solution onto a sample plate, and drying the dropped solution.

### EXAMPLES

### Synthesis of Matrices

The following 21 compounds 5, 10, 13, 16 to 21, 24 to 26, 29 to 32, 34 to 36, 41 and 42 were synthesized, wherein compounds 18 to 21, 25, 26, 41 and 42 are according to the present invention and compounds 5, 10, 13, 16, 17, 24, 29 to 32 and 34 to 36 are compounds used in the examples for comparative purposes and are not comprised by the present invention. In the chemical formula list shown below, 9-aminoacridine (9-AA) used as a target for comparison is illustrated together.

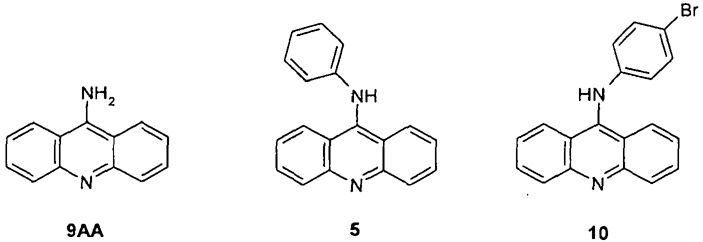

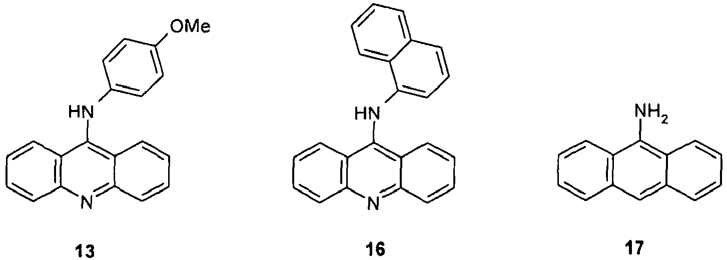

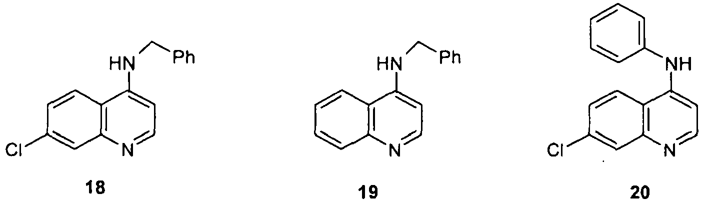

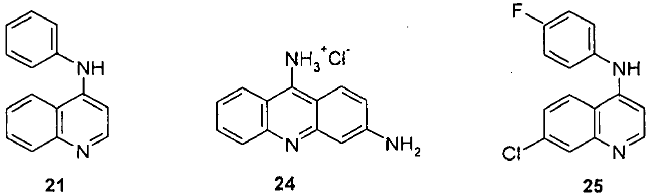

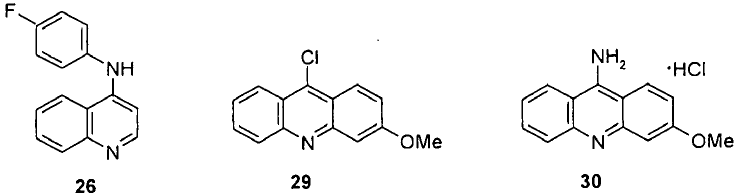

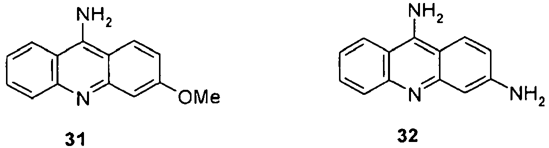

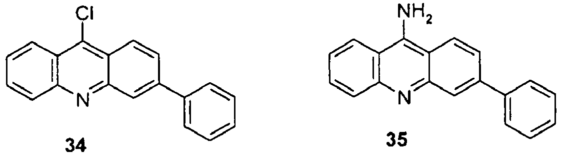

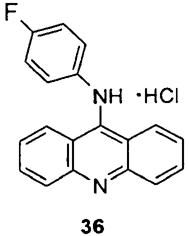

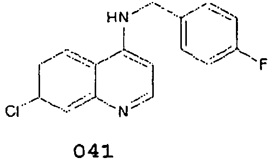

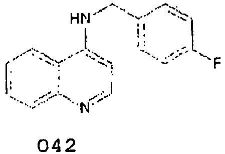

About compound 37 (anthracene) a commercially available product was used.

Synthesis of (9-phenylamino)acridine hydrochloride (4) and (9-phenylamino)acridine (5) which compounds are not comprised by the present invention:

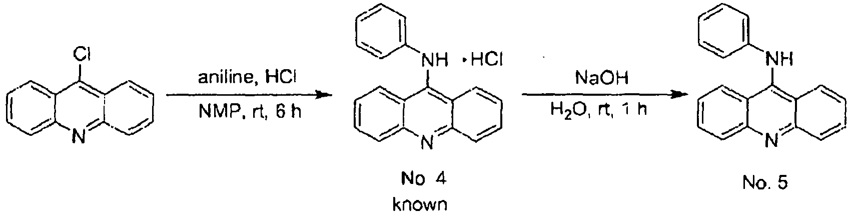

### Synthesis of (9-phenylamino)acridine hydrochloride (4):

Reference document: Cope, H. Mutter, R.; Heal, W.; Pascoe, C.; Brown, P.; Pratt, S.; Chen, B. Europian Journal of Medicinal Chemistry, 2006, 41, 1124-1143.

To a mixture of 9-chloroacridine (110 mg, 0.5 mmol), aniline (55.9 mg, 0.6 mmol, 1.2 equivalents), and 2.5 mL of 1-methyl-2-pyrrolidone (NMP), three drops of concentrated hydrochloric acid were added with a Pasteur pipette, and then the resultant was stirred at ambient temperature for 6 hours. Thereafter, thereto was added 20 mL of ethyl acetate, and then the resultant was stirred at ambient temperature for 1 hour. The precipitated crystal was suction-filtered while washed with ethyl acetate. Methanol and ethyl acetate were used to recrystallize the crystal. Yield: 104 mg, 68%.
¹H NMR (600 MHz, DMSO-d₆) δ: 7.39-7.45 (m, 5H), 7.49-7.53 (m, 2H), 8.01 (dd, J = 12, 12 Hz, 2H), 8.10 (d, J = 8.8 Hz, 2H), 8.24 (d, J = 8.8 Hz, 2H), ¹³C NMR (100 MHz, DMSO-d₆) δ: 113.62, 119.23, 123.74, 124.66, 125.76, 127.51, 129.96, 135.27, 140.09, 140.92, 155.24, MS (ESI)m/z : 271 (M+H)⁺

### Synthesis of (9-phenylamino)acridine (5):

Next, 63.5 mg of the hydrochloride salt and 120 mg of NaOH were added to 1.5 mL of water. The resultant was stirred for 1 hour, and then subjected to extraction with ethyl acetate. The extract was washed with water two times, washed with saturated sodium chloride solution, dried over sodium sulfate, and then concentrated. The resultant crystal was recrystallized with ethyl acetate. Yield: 38.0 mg, 57%; granular yellow crystal; m.p.: 227.2-228.9°C.

Synthesis of 9-(4-bromophenylamino)acridine (10) which compound is not comprised by the present invention:

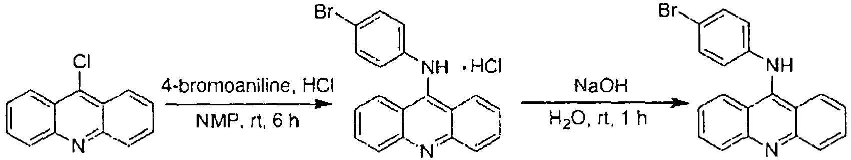

To a mixture of 9-chloroacridine (110 mg, 0.5 mmol), 4-bromoaniline (103 mg, 0.6 mmol, 1.2 equivalents), and 2.5 mL of NMP, three drops of concentrated hydrochloric acid were added with a Pasteur pipette, and then the resultant was stirred at ambient temperature for 6 hours. Thereafter, thereto was added 20 mL of ethyl acetate, and then the resultant was stirred at ambient temperature for 1 hour. The precipitated crystal was suction-filtered while washed with ethyl acetate. Methanol and acetonitrile were used to recrystallize the crystal. Yield: 127 mg, 66%; m.p.: 231.4°C.
¹H NMR (400 MHz, DMSO-d₆) δ: 7.41 (d, J = 8.8 Hz, 2H), 7.52 (t, J = 8.8 Hz, 2H), 7.71 (d, J = 8.8 Hz, 2H), 8.02-8.10 (m, 4H), 8.25 (d, J = 8.8 Hz, 2H), ¹³C NMR (100 MHz, DMSO-d₆) δ: 114.10, 114.94, 119.30, 123.98, 125.74, 126.06, 132.63, 135.32, 140.11, 140.71, 154.96; MS (ESI) m/z: 349 (M+H)⁺

Next, 77 mg of the hydrochloride salt and 120 mg of NaOH were added to 1.5 mL of water. The resultant was stirred for 1 hour, and then subjected to extraction with ethyl acetate. The extract was washed with water two times, washed with saturated sodium chloride solution, dried over sodium sulfate, and then concentrated. The resultant crystal was recrystallized with ethyl acetate. Yield: 36.3 mg, 52%; granular yellow crystal; m.p.: 220.7-221.9°C.

Synthesis of 9-(4-methoxyphenylamino)acridine (13) which compound is not comprised by the present invention:

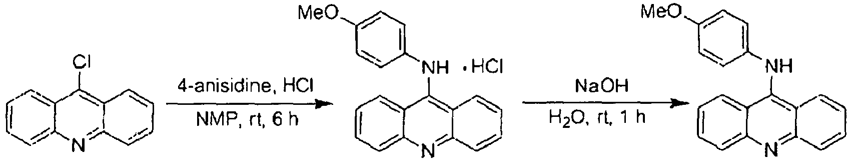

To a mixture of 9-chloroacridine (110 mg, 0.5 mmol), 4-anisidine (73.8 mg, 0.6 mmol, 1.2 equivalents), and 2.5 mL of NMP, three drops of concentrated hydrochloric acid were added with a Pasteur pipette, and then the resultant was stirred at ambient temperature for 6 hours. Thereafter, thereto was added 20 mL of ethyl acetate, and then the resultant was stirred at ambient temperature for 1 hour. The precipitated crystal was suction-filtered while washed with ethyl acetate. Yield (hydrochloride salt): 158 mg, 94%.
¹H NMR (400 MHz, DMSO-d₆) δ: 3.84 (s, 3H), 7.10 (d, J = 8.8 Hz, 2H), 7.42 (t, J = 8.8 Hz, 4H), 7.97 (t, J = 8.8 Hz, 2H), 8.10 (d, J = 8.8 Hz, 2H), 8.24 (d, J = 8.8 Hz, 2H), ¹³C NMR (100 MHz, DMSO-d₆) δ: 55.46, 113.15, 115.11, 119.06, 123.49, 125.65, 126.40, 133.21, 135.05, 140.02, 155.31, 158.53, MS (ESI) m/z : 301 (M+H)⁺

Next, 158 mg of the hydrochloride salt and 240 mg of NaOH were added to 3 mL of water. The resultant was stirred for 1 hour, and then subjected to extraction with ethyl acetate. The extract was washed with water two times, washed with saturated sodium chloride solution, dried over sodium sulfate, and then concentrated. The resultant crystal was recrystallized with water/methanol. Yield: 79.5 mg, 56%; dark red needles.

Synthesis of N-(4-methylphenyl)acridine-9-amine hydrochloride (14) and N-(4-methylphenyl)acridine-9-amine (36) which compounds are not comprised by the present invention:

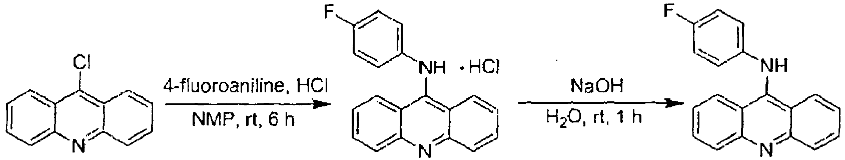

### Synthesis of 9-(4-fluorophenylamino)acridine hydrochloride (14):

To a mixture of 9-chloroacridine (110 mg, 0.5 mmol), 4-fluoroaniline (66.7 mg, 0.6 mmol, 1.2 equivalents), and 2.5 mL of NMP, three drops of concentrated hydrochloric acid were added with a Pasteur pipette, and then the resultant was stirred at ambient temperature for 6 hours. Thereafter, thereto was added 20 mL of ethyl acetate, and then the resultant was stirred at ambient temperature for 1 hour. The precipitated crystal was suction-filtered while washed with ethyl acetate. The crystal was recrystallized with acetonitrile. Yield: 125 mg, 77%; yellow needles.
¹H NMR (400 MHz, DMSO-d₆) δ: 7.38 (t, J = 8.8 Hz, 2H), 7.45 (t, J = 7.6 Hz, 2H), 7.51-7.55 (m, 2H), 7.99 (t, J = 7.6 Hz, 2H), 8.16 (d, J = 8.8 Hz, 2H), 8.26 (d, J = 8.8 Hz, 2H), ¹³C NMR (100 MHz, DMSO-d₆) δ: 113.49, 116.73 (d, J = 23 Hz), 119.18, 123.73, 125.73, 126.84 (d, J = 9.1 Hz), 135.16, 137.25, 140.08, 155.35, 160.73 (d, J = 246 Hz) ; MS (ESI) m/z: 289 (M+H)⁺

### Synthesis of 9-(4-fluorophenylamino)acridine (36):

To 1.5 mL of water were added 64.8 mg of the hydrochloride salt (14) and 120 mg of NaOH. The resultant was stirred for 1 hour, and then subjected to extraction with ethyl acetate. The extract was washed with water two times, washed with saturated sodium chloride solution, dried over sodium sulfate, and then concentrated. The resultant crystal was recrystallized with acetonitrile. Yield: 35.7 mg, 62%; yellow needles; m.p.: 183.5-196.1°C.

Synthesis of 9-(naphthalene-1-yl-amino)acridine (16) which compound is not comprised by the present invention:

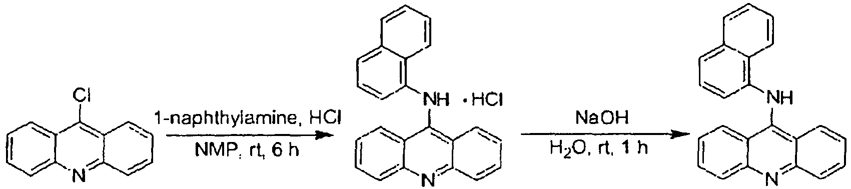

To a mixture of 9-chloroacridine (110 mg, 0.5 mmol), 1-naphthylamine (85.9 mg, 0.6 mmol, 1.2 equivalents), and 2.5 mL of NMP, three drops of concentrated hydrochloric acid were added with a Pasteur pipette, and then the resultant was stirred at ambient temperature for 6 hours. Thereafter, thereto was added 20 mL of ethyl acetate, and then the resultant was stirred at ambient temperature for 1 hour. The precipitated crystal was suction-filtered while washed with ethyl acetate. Yield (hydrochloride): 110 mg, 69%.
¹H NMR (400 MHz, DMSO-d₆) δ: 7.35 (t, J = 7.6 Hz, 2H), 7.59-7.70 (m, 4H), 7.98 (t, J = 7.6 Hz, 2H), 8.07-8.17 (m, 7H), MS (ESI) m/z: 321 (M+H)⁺

To 3 mL of water were added 110 mg of the hydrochloride salt and 240 mg of NaOH. The resultant was stirred for 1 hour, and then subjected to extraction with ethyl acetate. The extract was washed with water two times, washed with saturated sodium chloride solution, dried over sodium sulfate, and then concentrated.

Synthesis of 9-aminoanthracene (17) which compound is not comprised by the present invention:

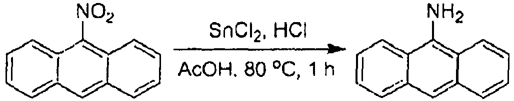

Reference document: Adams, H.; Bawa, R. A.; McMillan, K. G.; Jones, S. Tetrahedron: Asymmetry, 2007, 18, 1003-1012
9-Nitroantracene (446 mg, 2.00 mmol) was added to acetic acid (9.6 g, 160 mmol, 80 equivalents), and the resultant was stirred at 70°C for 1 hour. Thereto was slowly added a solution obtained by dissolving SnCl₂ (1.89 g, 10 mmol, 5 equivalents) in concentrated hydrochloric acid (7.3 g, 200 mmol, 100 equivalents), and the resultant was stirred at 80°C for 1 hour. The precipitated crystal was then suction-filtered while washed with concentrated hydrochloric acid. Thereafter, the filtrate was added to 30 mL of a 10% NaOH aqueous solution, and the resultant was stirred for 1 hour. The resultant was then suction-filtered while washed with water. The resultant crude crystal was recrystallized with methanol. Yield: 270 mg, 70%; reddish purple needles; m.p.: 137.9-171.2°C.
¹H NMR (400 MHz, CDCl₃) δ: 4.87 (s, 2H), 7.39 - 7.46 (m, 4H), 7.88 (s, 1H), 7.88 - 7.98 (m, 4H), MS (ESI) m/z: 194 (M+H)⁺

Synthesis of 4-(N-benzyl)amino-7-chloroquinoline (18) which compound is comprised by the present invention:

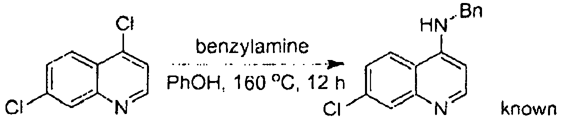

Reference documents: Pellegrini, S.; Grad, J-N.; Bousquet, T.; Pelinski, L. Tetrahedron Lett. 2011, 52, 1742-1744, and
de Souza, M. V. N.; Pais, K. C.; Kaiser, C. R.; Peralta, M. A.; Ferreira, M. de L.; Lourenco, M. C. S. Bioorganic and Medicinal Chemistry, 2009, 17, 1474-1480.

To 25 mL of phenol was added 4,7-dichloroquinoline (1.98 g, 10 mmol), and then the resultant was stirred at 120°C. Thereafter, the temperature thereof was raised to 160°C, and thereto was added benzylamine (1.61 g, 15 mmol, 1.5 equivalents). The resultant was stirred for 12 hours, and then the temperature thereof was returned to ambient temperature. Thereto was added 30 mL of acetone, and the temperature thereof was set to 0°C. The resultant was stirred for 1 hour. The precipitated crystal was then suction-filtered while washed with acetone. The resultant crystal was added to 100 mL of a 10% NaOH aqueous solution. The resultant was stirred for 1 hour, and subjected to extraction with chloroform. The extract was washed with water two times, washed with saturated sodium chloride solution, dried over sodium sulfate, and then concentrated. The crystal was recrystallized with ethyl acetate. Yield: 552 mg, 21%; colorless needles; m.p.: 173.1-174.6°C.
¹H NMR (400 MHz, CDCl₃) δ: 4.53 (d, J = 5.2 Hz, 2H), 5.32 (s, 1H), 6.46 (d, J = 6.0 Hz, 1H), 7.34 - 7.40 (m, 6H), 7.69 (d, J = 8.8 Hz, 1H), 7.98 (d, J = 2.0 Hz, 1H), 8.53 (d, J = 4.8 Hz, 1H), ¹³C NMR (100 MHz, CDCl₃) δ: 47.61, 99.70, 117.13, 120.84, 125.50, 127.59, 127.98, 129.01, 134.94, 137.19, 149.17, 149.41, 152.14, MS (ESI) m/z : 269 (M+H)⁺

Synthesis of 4-(N-benzylamino)quinoline (19) which compound is comprised by the present invention:

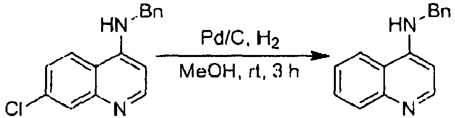

Reference document: Masatomo Hamana, Kazuhisa Funakoshi, Yakugaku Zasshi, 1964, 84, 42-47.

4-(N-benzyl)amino-7-chloroquinoline (485 mg, 1.80 mmol) was dissolved in 12 mL of methanol. Thereto was added Pd/C (10%, 25.5 mg, 0.024 mmol, 0.01 equivalents). Hydrogen was added to the resultant while the system was bubbled therewith at ambient temperature under normal pressure. The resultant was stirred for 3 hours. Thereafter, the resultant was filtered through celite, and concentrated. Next, thereto was added 30 mL of a 10% NaOH aqueous solution. The resultant was stirred for 1 hour, and subjected to extraction with chloroform. The extract was washed with water two times, washed with saturated sodium chloride solution, dried over sodium sulfate, and concentrated. The crystal was recrystallized with acetonitrile. Yield: 335 mg, 66%; colorless needles; m.p.: 131.5-132.2°C (bibliographic data: 113-115°C (benzene/petroleum benzine)).
¹H NMR (400 MHz, CDCl₃) δ: 4.54 (d, J = 4.8 Hz, 2H), 5.40 (s, 1H), 6.46 (d, J = 4.8 Hz, 1 H), 7.35 - 7.45 (m, 6H), 7.64 (t, J = 7.6 Hz, 1 H), 7.77 (d, J = 8.8 Hz, 1H), 8.00 (d, J = 8.8 Hz, 1H), 8.55 (d, J = 5.2 Hz, 1H), ¹³C NMR (100 MHz, CDCl₃-d6) δ: 47.55, 99.39, 118.73, 119.26, 124.75, 127.54, 127.82, 128.93, 129.03, 130.07, 137.52, 148.46, 149.39, 151.11, MS (ESI) m/z : 235 (M+H)⁺

Synthesis of 7-chloro-4-(N-phenylamino)quinoline (20) which compound is comprised by the present invention:

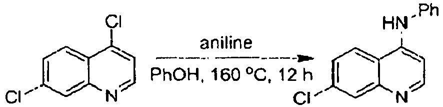

Reference documents: Chambers, R. A.; Pearson, D. E. J Org. Chem. 1963, 28, 3144-3147, and
Souza, M. Bioorganic and Medicinal Chemistry, 2009, 17, 1474-1480.

To 25 mL of phenol was added 4,7-dichloroquinoline (1.98 g, 10 mmol), and then the resultant was stirred at 120°C. Thereafter, the temperature thereof was raised to 160°C, and thereto was added aniline (1.40 g, 15 mmol, 1.5 equivalents). The resultant was then stirred for 12 hours, and then the temperature thereof was returned to ambient temperature. Thereto was added acetone, and the temperature thereof was set to 0°C. The resultant was stirred for 1 hour. The precipitated crystal was then suction-filtered while washed with acetone. The resultant crystal was added to 100 mL of a 10% NaOH aqueous solution. The resultant was stirred for 1 hour, and subjected to extraction with chloroform. The extract was washed with water two times, washed with saturated sodium chloride solution, dried over sodium sulfate, and then concentrated. The crystal was recrystallized with acetonitrile. Yield: 888 mg, 35%; granular colorless crystal.
¹H NMR (400 MHz, DMSO-d₆) δ: 6.92 (d, J = 6.0 Hz, 1H), 7.17 (t, J = 7.6 Hz, 1H), 7.36 - 7.46 (m, 4H), 7.58 (dd, J = 2.0 , 7.6 Hz, 1H), 7.90 (d, J = 2.0 Hz, 1H), 8.43 - 8.47 (m, 2H), 9.10 ppm (s, 1H), ¹³C NMR (100 MHz, DMSO) δ: 101.71, 118.29, 122.64, 124.04, 124.42, 124.88, 127.65, 129.40, 133.85, 140.11, 147.95, 149.58, 151.95, MS (ESI) m/z : 255 (M+H)⁺

Synthesis of 4-(N-phenylamino)quinoline (21) which compound is comprised by the present invention:

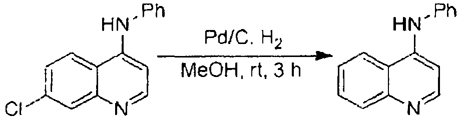

Reference documents: Alan R. Katritzky, A. R.; Tian-Bao Huang, T-B.; Voronkov, M. V. J. Org. Chem. 2001, 66, 1043-1045, and
Souza, M. Bioorganic and Medicinal Chemistry, 2009, 17, 1474-1480.

In 30 mL of methanol was dissolved N-phenyl-7-chloroquinoline-4-amine (180 mg, 0.709 mmol). Thereto was added Pd/C (10%, 10 mg, 0.0009 mmol, 0.013 equivalents). Hydrogen was added to the resultant while the system was bubbled therewith at ambient temperature under normal pressure. The resultant was stirred for 3 hours. Thereafter, the resultant was filtered through celite, and concentrated. Next, thereto was added 10 mL of a 10% NaOH aqueous solution. The resultant was stirred for 1 hour, and subjected to extraction with chloroform. The extract was washed with water two times, washed with saturated sodium chloride solution, dried over sodium sulfate, and concentrated. The resultant was recrystallized with acetonitrile. Yield: 57.7 mg, 37%; granular light yellow crystal; m.p.: 194.8-195.6°C (bibliographic data m.p.: 197-198°C).
¹H NMR (400 MHz, CDCl₃) δ: 6.77 (s, 1H), 7.00 (d, J = 5.2 Hz, 1H), 7.19, (t, J = 6.8 Hz, 1 H) 7.31 (d, J = 8.8 Hz, 2H), 7.42 (t, J = 8.0 Hz, 2H), 7.50 (t, J = 8.0 Hz, 1H), 7.69 (t, J = 6.8 Hz, 1H), 7.95 (d, J = 8.8 Hz, 1H), 8.05 (d, J = 8.8 Hz, 1H) 8.58 (d, J = 5.2 Hz, 1H), ¹³C NMR (100 MHz, CDCl₃) δ: 102.23, 119.57, 119.71, 122.60, 124.59, 125.32, 129.34, 129.68, 130.19, 139.87, 147.40, 149.13, 150.93, MS (ESI) m/z : 221 (M+H)⁺

Synthesis of 7-chloro-N-(4-fluorophenylamino)quinoline (25) which compound is comprised by the present invention:

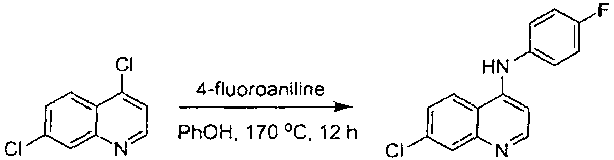

Reference document: Motiwala, F. Australian Journal of Chemistry 2007, 60, 369-374
4,7-dichloroquinoline (1.98 g, 10 mmol) was added to 25 mL of phenol. The resultant was stirred at 120°C, and then the temperature thereof was raised to 170°C. Thereto was added 4-fluoroaniline (1.67 g, 15 mmol, 1.5 equivalents), and then the resultant was stirred for 12 hours. The resultant was then cooled to ambient temperature, and thereto was added 30 mL of acetone. The temperature of the system was set to 0°C, and then the resultant was stirred for 1 hour. The precipitated crystal was suction-filtered while washed with acetone. Next, the filtrate was added to 100 mL of a 10% NaOH aqueous solution, and the resultant was stirred for 1 hour. The resultant was subjected to extraction with chloroform. The extract was washed with water two times, washed with saturated sodium chloride solution, dried over sodium sulfate, and concentrated. The crystal was recrystallized with acetonitrile. Yield: 1.35 g, 50%; granular purple crystal.

¹H NMR (400 MHz, DMSO-d₆) δ: 6.78 (d, J = 5.2 Hz, 1H), 7.28 (t, J = 8.8 Hz, 2H), 7.39 (dd, J = 5.2, 8.8 Hz, 2H), 7.58 (dd, J = 2.0 , 9.6 Hz, 1H), 7.90 (d, J = 2.0 Hz, 1H), 8.40 - 8.46 (m, 2H), 9.08 (broad, 1H), ¹³C NMR (100 MHz, DMSO-d₆) δ: 101.24, 116.13 (d, J = 22 Hz), 118.04, 124.59 (d, J = 59 Hz), 125.27 (d, J = 8.2 Hz), 127.66, 133.86, 136.27, 148.39, 148.50, 151.96, 158.89 (d, J = 242 Hz), 168.25, MS (ESI) m/z : 273 (M+H)⁺

Synthesis of N-(4-fluorophenyl)quinoline-4-amine (26) which compound is comprised by the present invention:

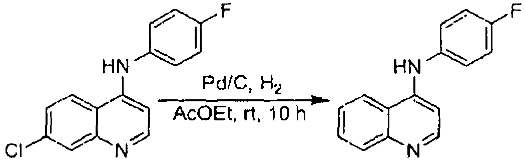

7-chloro-N-(4-fluorophenyl)quinoline-4-amine (1.29 g, 4.74 mmol) was dissolved in 30 mL of ethyl acetate. Thereto was added Pd/C (10%, 65.6 mg, 0.0616 mmol, 0.013 equivalents). Hydrogen was added to the resultant while the system was bubbled therewith at ambient temperature under normal pressure. The resultant was stirred for 10 hours. Thereafter, the resultant was filtered through celite, and concentrated. Next, thereto was added 100 mL of a 10% NaOH aqueous solution. The resultant was stirred for 1 hour, and subjected to extraction with chloroform. The extract was washed with water two times, washed with saturated sodium chloride solution, dried over sodium sulfate, and concentrated. The crystal was recrystallized with acetonitrile. Yield: 662 mg, 59%; granular colorless crystal.
¹H NMR (400 MHz, DMSO-d₆) δ: 6.79 (d, J = 4.8 Hz, 1H), 7.27 (t, J = 8.8 Hz, 2H)7.40 (dd, J = 4.8, 8.8 Hz, 2H), 7.53 (t, J = 8.0 Hz, 2H), 7.70 (t, J = 8.0 Hz, 1H), 7.88 (d, J = 8.0 Hz, 1H), 8.37 (d, J = 8.8 Hz, 2H), 8.44 (d, J = 6.0 Hz, 1H), 8.93 ppm (broad, 1H), ¹³C NMR (100 MHz, DMSO-d₆) δ: 100.94, 115.95, 116.17, 119.48, 121.95, 124.56, 125.00 (d, J = 8.2 Hz), 129.17, 136.72, 148.09, 148.84, 150.66, 158.69 (d, J = 241 Hz), MS (ESI) m/z : 239 (M+H)⁺

Synthesis of 3-methoxy-9-aminoacridine (29) which compound is not comprised by the present invention:

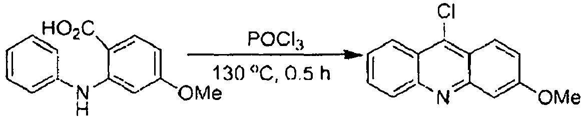

Phosphorous oxychloride (8.09 g, 52.8 mmol, 22 equivalents) was added to 4-methoxy-2-(phenylamino)benzoic acid (584 mg, 2.4 mmol). The resultant was stirred at 130°C for 30 minutes. Thereafter, the resultant was cooled to ambient temperature, and then thereto was added a 28% ammonia aqueous solution until the system became basic. The resultant was subjected to extraction with chloroform. The extract was washed with water two times, washed with saturated sodium chloride solution, dried over sodium sulfate, and concentrated. The resultant crude crystal was recrystallized with methanol. Yield: 391 mg, 67%; light yellow; m.p.: 169.4-169.5°C.
¹H NMR (400 MHz, CDCl₃) δ: 4.01 (s, 3H), 7.30 (d, J = 10 Hz, 1H), 7.43 (s, 1H), 7.58(t ,J = 8.8 Hz, 1H), 7.79 (t, J = 8.0 Hz, 1H), 8.14 (d, J = 8.8 Hz, 1H), 8.31 (d, J = 9.6 Hz, 1H), 8.39 (d, J = 8.8 Hz, 1H), ¹³C NMR (100 MHz, CDCl₃) δ: 55.65, 105.24, 120.39, 122.24, 123.13, 124.63, 125.74, 125.80, 128.99, 130.49, 140.88, 149.11, 150.66, 161.50, MS (EI) m/z : 243 (M+H)⁺

### Synthesis of 3-methoxy-9-aminoacridine (31) which compound is not comprised by the present invention:

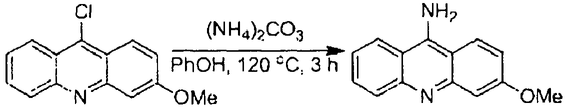

Phenol (1.02 g, 10.8 mmol, 10 equivalents) was added to 3-methoxy-9-chloroacridine (262 mg, 1.08 mmol). The resultant was stirred at 70°C for 1 hour. Thereafter, thereto was added ammonium carbonate (207 mg, 2.16 mmol, 1.5 equivalents). The temperature of the resultant was raised to 120°C, and then the resultant was stirred for 3 hours. The temperature was returned to ambient temperature, and then thereto was added acetone. The temperature was set to 0°C, and the resultant was stirred for 1 hour. Next, thereto was added 15 mL of 2.5 M NaOH, and the resultant was stirred for 1 hour. The resultant was subjected to extraction with ethyl acetate. The extract was washed with water two times, washed with saturated sodium chloride solution, dried over sodium sulfate, and concentrated. The crystal was recrystallized with methanol. Yield: 194 mg, 80%; granular yellow crystal; m.p.: 196.9-199.8°C.
¹H NMR (400 MHz, DMSO-d₆) δ: 3.92 (s, 3H), 6.99 (d, J = 8.8 Hz, 1H), 7.17 (s, 1H), 7.27 (t, J = 7.6 Hz, 1H), 7.62 (t, J = 8.8 Hz, 1H), 7.69 (broad, 2H), 7.77 (d, J = 8.8 Hz, 1H), 8.31-8.37 ppm (m, 2H), ¹³C NMR (100 MHz, DMSO-d₆) δ: 55.17, 105.45, 108.16, 112.78, 115.41, 120.87, 123.27, 124.81, 128.17, 129.77, 149.19, 149.82, 150.86, 160.62 ppm, MS (ESI) m/z : 225 (M+H)⁺

Synthesis of 3-phenyl-9-chloroacridine (34) which compound is not comprised by the present invention:

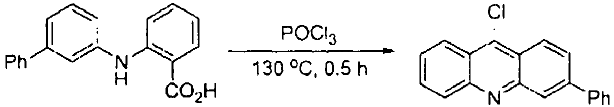

Phosphorous oxychloride (11.5 g, 75 mmol, 25 equivalents) was added to 2-([1,1'-biphenyl]-3-yl-amino)benzoic acid (868 mg, 3 mmol). The temperature of the resultant was set to 130°C, and the resultant was stirred at 130°C for 30 minutes. Thereafter, the resultant was cooled to ambient temperature, and then thereto was added a 28% ammonia aqueous solution until the system became basic. The resultant was subjected to extraction with chloroform. The extract was washed with water two times, washed with saturated sodium chloride solution, dried over sodium sulfate, and concentrated. The crystal was recrystallized with acetonitrile. Yield: 593 mg, 68%; light yellow needles; m.p.: 104.0-107.9°C.
¹H NMR (400 MHz, DMSO-d₆) δ: 7.36-7.38 (m, 2H), 7.54 (t, J = 6.8 Hz, 2H), 7.57-7.66 (m, 2H), 7.75-7.85 (m, 5H), 7.95 (dd, J = 2.0, 8.8 Hz, 1H), ¹³C NMR (100 MHz, DMSO-d₆) δ: 124.70, 125.00, 125.23, 126.82, 126.85, 126.97, 127.02, 127.45, 127.69, 128.37, 129.12, 129.45, 129.55, 129.78, 130.16, 130.61, 130.67, MS (ESI) m/z : 290 (M+H)⁺

Synthesis of 3-phenyl-9-aminoacridine (35) which compound is not comprised by the present invention:

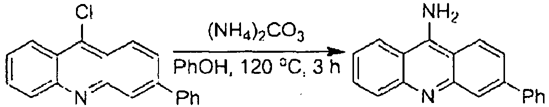

Phenol (941 mg, 10 mmol, 10 equivalents) was added to 3-phenyl-9-chloroacridine (290 mg, 1.00 mmol). The resultant was stirred at 70°C for 1 hour. Thereafter, thereto was added ammonium carbonate (192 mg, 2.00 mmol, 2 equivalents), and then the temperature of the resultant was raised to 120°C. The resultant was then stirred for 3 hours. The temperature was returned to ambient temperature, and then thereto was added acetone. The temperature was set to 0°C, and the resultant was stirred for 1 hour. Next, thereto was added 10 mL of 2.5 M NaOH, and the resultant was stirred for 1 hour and then subjected to extraction with ethyl acetate. The extract was washed with water two times, washed with saturated sodium chloride solution, dried over sodium sulfate, and concentrated. The crystal was recrystallized with acetonitrile. Yield: 198 mg, 73%; granular yellow crystal.
¹H NMR (400 MHz, DMSO-d₆) δ: 7.53-7.62 (m, 4H), 7.86-8.02 (m, 5H), 8.21 (s, 1H), 8.75 (d, J = 8.8 Hz, 1H), 8.83 (d, J = 8.8 Hz, 1H), 10.13 (broad, 2H), ¹³C NMR (100 MHz, DMSO-d₆) δ: 110.68, 111.70, 115.44, 118.71, 122.81, 123.77, 124.88, 125.78, 127.24, 129.35, 135.48, 137.89, 139.58, 139.88, 146.44, 157.42, MS (ESI) m/z : 271 (M+H)⁺

Synthesis of 4-(N-p-fluorobenzyl)amino-7-chloroquinoline (41) which compound is comprised by the present invention:

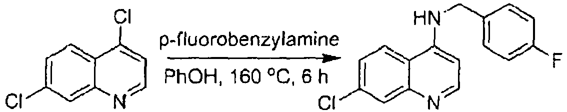

4,7-Dichloroquinoline (1.98 g, 10 mmol) was added to 25 mL of phenol. The resultant was stirred at 120°C, and then the temperature was raised to 160°C. Thereto was then added p-fluorobenzylamine (1.61 g, 15 mmol, 1.5 equivalents). The resultant was stirred for 6 hours, and the temperature was returned to ambient temperature. Thereto was added 30 mL of acetone. The temperature of the system was set to 0°C, and then the resultant was stirred for 1 hour. The precipitated crystal was suction-filtered while washed with acetone. The resultant crystal was added to 100 mL of a 10% NaOH aqueous solution, and the resultant was stirred for 1 hour. The resultant was subjected to extraction with chloroform. The extract was washed with water two times, washed with saturated sodium chloride solution, dried over sodium sulfate, and concentrated. The crystal was recrystallized with acetonitrile. Yield: 1.52 g, 53%; colorless needles; m.p.: 194.9-196.0°C.
IR (KBr) 3217 (NH) cm⁻¹; ¹H NMR (400 MHz, DMSO-d₆) δ: 4.52 (d, J = 5.9 Hz, 2H), 6.34 (d, J = 5.9 Hz, 1H), 7.14 (t, J = 8.8 Hz), 7.41 - 7.49 (m, 3H), 7.79 (s, 1H), 8.03 (s, 1H), 8.31 - 8.33 (m, 2H), ¹³C NMR (100 MHz, DMSO-d₆) δ: 47.79, 99.41, 115.06, 115.28, 117.51, 123.97, 124.30, 127.57, 128.83, 128.92, 133.45, 134.69, 134.71, 149.04, 149.78, 151.79, 159.99, 162.43; MS (EI) m/z : 286 (M+H)⁺

Synthesis of 4-(N-p-fluorobenzylamino)quinoline (42) which compound is comprised by the present invention:

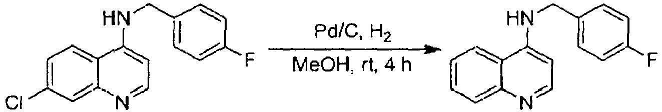

4-(N-9-fluorobenzyl)amino-7-chloroquinoline (41) (287 mg, 1 mmol) was dissolved in 20 mL of methanol. Thereto was added Pd/C (10%, 11 mg, 0.01 mmol, 0.01 equivalents). Hydrogen was added to the resultant while the system was bubbled therewith at ambient temperature under normal pressure. The resultant was stirred for 4 hours. Thereafter, the resultant was filtered through celite, and concentrated. Next, thereto was added 20 mL of a 10% NaOH aqueous solution. The resultant was stirred for 1 hour, and subjected to extraction with chloroform. The extract was washed with water two times, washed with saturated sodium chloride solution, dried over sodium sulfate, and concentrated. The crystal was recrystallized with acetonitrile. Yield: 179 mg, 71%; colorless needles; m.p.: 180.3-181.7°C.
IR (KBr) 3221 (NH) cm⁻¹; ¹H NMR (400 MHz, DMSO-d₆) δ: 4.53 (d, J = 5.8 Hz, 2H), 6.32 (d, J = 4.9 Hz, 1H), 7.14 (t, J = 8.8Hz), 7.40 - 7.46 (m, 3H), 7.61 (t, J = 8.8 Hz, 1H), 7.78 (d, J = 7.8 Hz, 1H), 7.89 (s, 1H), 8.26 - 8.31 (m, 2H), ¹³C NMR (100 MHz, DMSO-d₆) δ: 44.79, 98.95, 115.03, 115.24, 118.90, 121.57, 124.00, 128.75, 128.83, 129.09, 134.97, 135.01, 148.28, 149.57, 150.54, 159.95, 162.38; MS (EI) m/z : 252 (M+H)⁺

### Example (1) of spectrum analysis:

Six samples were prepared, in each of which plural anionic compounds were mixed with each other (Tables 1 to 6 shown below). Each of these mixtures was subjected to MALDI mass spectrometry in a negative ion mode to evaluate an effect of each of the matrices that was produced on the ability of ionizing each of the anionic compounds and on the peak strength thereof. Each of the mixtures was mixed with the matrix at a ratio selected at will. The mixture was naturally dried on a stainless steel plate for MALDI. This sample was measured using a MALDI mass spectrometer (MALDI-TOF-MS: AXIMA, Performance, manufactured by Shimadzu Corp.).

**[Table 1]**

| Compound | m/z |
|---|---|
| 3,4-Dihydroxyphenylacetic acid | 167.035 |
| Acetic acid 4-hydroxyphenylacetic acid | 151.0401 |
| 4-Hydroxyphenylpyruvic acid | 179.035 |
| 5-Hydroxyindoleacetic acid | 190.051 |
| N-acetyl-aspartyl-glutamic acid (NAAG) | 303.0834 |
| N-acetylcysteine | 162.023 |
| N-acetylglutamine | 187.0724 |
| N-acetylglycine | 116.0353 |
| N-acetylphenylalanine | 206.0823 |
| N-acetyltyrosine | 250.1085 |
| Alanine | 88.04041 |
| Anthranilic acid | 136.0404 |
| Asparagine | 131.0462 |
| Aspartic acid | 132.0302 |
| β-Hydroxyisovaleric acid | 117.0557 |
| Betainealdehyde | 101.0846 |
| Cysteine | 120.0125 |
| Glutamine | 145.0619 |
| Glutaric acid (pentanedicarboxylic acid) | 131.035 |
| Glycine | 74.02476 |
| Histamine | 110.0724 |
| Histidine | 154.0622 |
| Isoleucine | 130.0874 |
| Ornithine | 131.0826 |
| Phenylacetylglycine | 192.0666 |
| Phenylalanine | 164.0717 |
| Pipecolic acid | 128.0717 |
| Serine | 104.0353 |
| Threonine | 118.051 |
| Tryptophan | 203.0826 |
| Tyramine | 136.0768 |
| Tyrosine | 180.0666 |
| Urocanic acid | 137.0357 |
| Valine | 148.0438 |
| Xanthurenic acid | 204.0302 |

**[Table 2]**

| Compound | m/z |
|---|---|
| 1,5-Anhydroglucitol (1,5-AG) | 163.0612 |
| 2'-Deoxyinosine | 251.0786 |
| 5-Aminovaleric acid | 116.0717 |
| 5-Methylcytidine | 256.0939 |
| 5-Methylcytosine | 124.0516 |
| 5-Oxoproline | 128.0353 |
| Adenosine | 266.0895 |
| Agmatine | 129.1146 |
| Citric acid | 191.0197 |
| Cysteine-glutathionedisulfide | 425.0806 |
| Cytidine | 242.0783 |
| Erythrose | 119.035 |
| Fructose | 179.0561 |
| Fumaric acid | 115.0037 |
| Gluconic acid | 195.051 |
| Glutathione, oxidized type (GSSG) | 611.1447 |
| Glutathione, reduced type (GSH) | 306.0765 |
| Inosine | 267.0735 |
| Itaconic acid (methylenesuccinic acid) | 129.0193 |
| Lactic acid | 89.02442 |
| Maltopentaose | 827.2674 |
| Maltose | 341.1089 |
| Maltotetraose | 665.2146 |
| Maltotriose | 503.1618 |
| Phosphoric acid | 96.96963 |
| Phosphoenolpyruvic acid (PEP) | 166.9751 |
| Proline | 114.0561 |
| Ribose 5-phosphoric acid | 229.0119 |
| Sarcosine (N-methylglycine) | 88.04041 |
| Succinic acid | 117.0193 |
| Thymidine | 241.083 |
| Thymine | 157.0077 |
| Urea | 59.02509 |
| Xanthine | 151.0262 |

**[Table 3]**

| Compound | m/z |
|---|---|
| 5-Methyltetrahydrofolic acid (5MeTHF) | 458.1793 |
| Acetylcarnitine | 203.1163 |
| Adipic acid | 145.0506 |
| Adrenic acid (22:4n6) | 331.2643 |
| α-Tocopherol | 429.3738 |
| Ascorbic acid (vitamin C) | 175.0248 |
| Azelaic acid (nonanedicarboxylic acid) | 187.0976 |
| Biliverdin | 581.2406 |
| Caproic acid (6:0) | 115.0765 |
| Caprylic acid (8:0) | 143.1078 |
| Choline | 102.0924 |
| Ethanolamine | 60.04549 |
| Flavin adenine dinucleotide (FAD) | 784.1499 |
| Glycerol | 91.04007 |
| Hem | 615.17 |
| Heptanoic acid (7:0) | 129.0921 |
| Isovaleric acid | 101.0608 |
| lauric acid (12:0) | 199.1704 |
| Linolic acid (18:2n6) | 279.233 |
| Linolenic acid | 277.2173 |
| Methyl palmitate | 269.2486 |
| Myristic acid (14:0) | 227.2017 |
| Myristoleic acid (14:1n5) | 225.186 |
| Nicotinic acid | 122.0248 |
| Palmitoleic acid (16:1n7) | 253.2173 |
| Pentadecanoic acid (15:0) | 241.2173 |
| Phosphoethanolamine | 140.0118 |
| Quinolinic acid | 166.0146 |
| Sebacic acid (decanedicarboxylic acid) | 201.1132 |
| Stearic acid (18:0) | 283.2643 |
| Thiamine (vitamin B1) | 263.0972 |
| Uracil | 111.02 |
| Uridine | 243.0623 |

**[Table 4]**

| Compound | m/z |
|---|---|
| 3-Hydroxylactic acid (BHBA) | 103.0401 |
| 5-Aminolevulinic acid | 130.0509 |
| 5-Methyl-2'-deoxycitidine | 240.099 |
| 7-Dehydrocholesterol | 383.3319 |
| ATP | 505.9885 |
| Acetyl-CoA | 808.1185 |
| CDP | 402.0109 |
| CTP | 481.9772 |
| Cytosine | 110.036 |
| D-alanyl-D-alanine | 159.0775 |
| Deoxyadenosine | 250.0946 |
| Deoxyguanosine | 266.0895 |
| Folic acid | 438.1167 |
| GTP | 521.9834 |
| Guanosine | 282.0844 |
| IMP | 347.0398 |
| L-homocysteine | 134.0281 |
| L-homoserine | 118.0509 |
| Pregnenolone | 315.2329 |
| Spermine | 201.2084 |
| Stigmasterol | 411.3632 |
| Testosterone | 287.2016 |
| UDP | 402.9949 |
| UTP | 482.9612 |
| β-Sitosterol | 413.3789 |
| Cholic acid | 407.2803 |
| Cholesterol | 385.3476 |
| Corticosterone | 345.2071 |
| dATP | 489.9935 |
| dCMP | 306.0496 |
| dGDP | 426.0221 |
| Hyodeoxycholic acid | 391.2854 |
| Mevalonic acid | 147.0663 |
| myo-Inositol | 179.0561 |
| Sphingosine | 298.2752 |

**[Table 5]**

| Compound | m/z |
|---|---|
| 1-(5'-Phosphoribosyl)-5-amino-4-imidazolecarboxamide | 337.0554 |
| 1-Aminocyclopropane-1-carboxylic acid | 100.0404 |
| 3-Hydroxyoctanoic acid | 159.1026 |
| 4-Aminobenzoic acid | 136.0404 |
| 4-Coumaric acid | 163.04 |
| Benzoic acid | 121.0295 |
| Serotetraose | 665.2146 |
| D-mannitol | 181.0718 |
| D-xylose | 149.0455 |
| Deoxycholic acid | 391.2854 |
| Diethanolamine | 104.0717 |
| Ethylmalonic acid | 131.035 |
| Homogentisic acid | 167.035 |
| L-methionine-S-oxide | 164.0387 |
| Malic acid | 133.0142 |
| Maleic acid | 115.0037 |
| Monomethyl glutarate | 145.0506 |
| Nicotinuric acid | 179.0462 |
| O-acetyl-L-homoserine | 160.0615 |
| Quinaldic acid | 172.0404 |
| Sedoheptulose | 209.0667 |
| Thiaminediphosphoric acid | 424.0377 |
| UDP-glucose | 565.0477 |
| cis-Cinnamic acid | 147.0454 |
| γ-Butyrolactone | 85.02953 |
| Glucose | 179.0561 |

**[Table 6]**

| Compound | m/z |
|---|---|
| 2-Aminobutyric acid | 102.0561 |
| 3,4-Dihydroxyphenylacetic acid | 167.035 |
| 3-(3-Hydroxyphenyl)propionic acid | 165.0557 |
| 3-Hydroxydecanoic acid | 187.1339 |
| 3-Hydroxyphenylacetic acid | 151.0401 |
| 3-Methyladipic acid | 159.0663 |
| 3-Methylhistidine | 168.0779 |
| L-hydroxyproline | 130.0509 |
| N-acetylleucine | 172.0979 |
| N-acetylmethionine | 190.0543 |
| N-acetylproline | 156.0666 |
| Pyridoxamine | 167.0826 |
| Allantoin | 157.0367 |
| Arabinose | 149.0456 |
| Arginine | 173.1044 |
| β-D-fructose 6-phosphate | 259.0224 |
| β-Alanine | 88.04041 |
| Capric acid (10:0) | 171.1391 |
| Ciliatine (2-aminoethyl phosphonic acid) | 124.0169 |
| Glutamic acid | 146.0459 |
| Homocitrulline | 188.104 |
| Leucine | 130.0874 |
| Lysine | 145.0983 |
| Mannose | 179.0561 |
| Margaric acid (17:0) | 269.2486 |
| Methionine | 148.0438 |
| Palmitic acid (16:0) | 255.233 |
| Pseudouridine | 243.0623 |
| Putrescine | 87.09278 |
| Raffinose | 503.1618 |
| Ribose | 149.0456 |
| scyllo-Inositol | 179.0561 |
| Spermidine | 144.1506 |
| Stachyose | 665.2146 |
| Sucrose | 341.1089 |
| Valeric acid | 101.0608 |
| Xylitol | 151.0612 |
| Xylonic acid | 165.0405 |

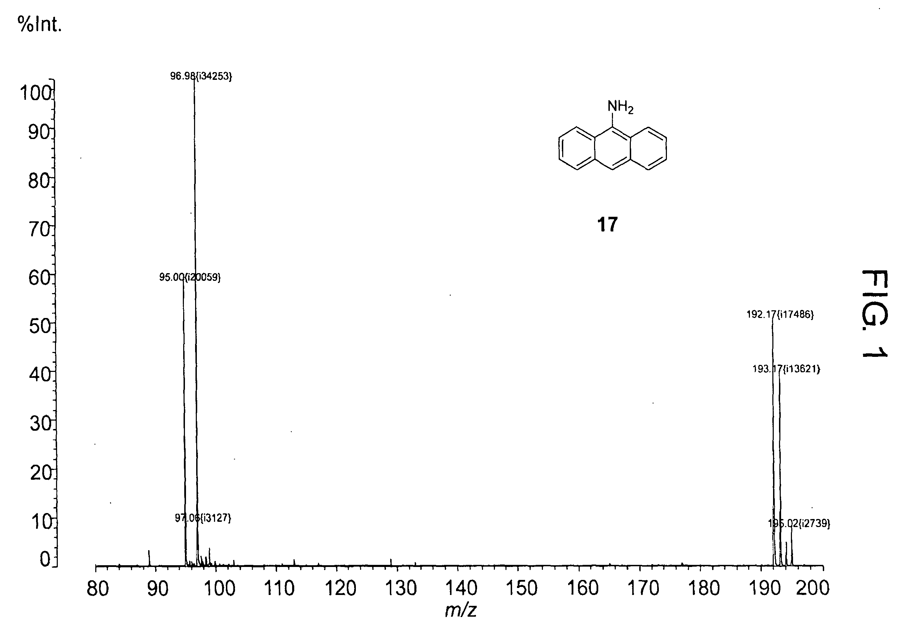

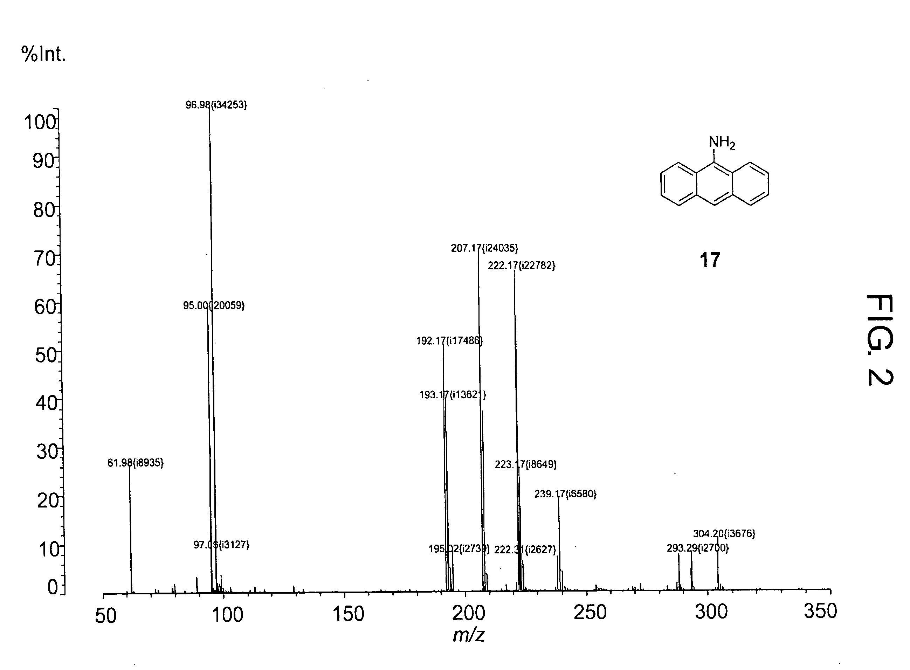

Figs. 1 to 3 each show a result obtained by using 9-aminoanthracene (17) as a matrix to make MALDI mass spectrometry in a negative ion mode which compound is not comprised by the present invention and is used for comparative purposes only. Figs. 1 and 2 are each a mass spectrum showing a result of the measurement of a blank containing no sample. A peak (m/z: 192) of a proton-desorbed ion [M-H]⁻ of the matrix, and a peak (m/z: 193) of a M- ion are observed. Other peaks are peaks which originate from the matrix and are unable to be assigned.

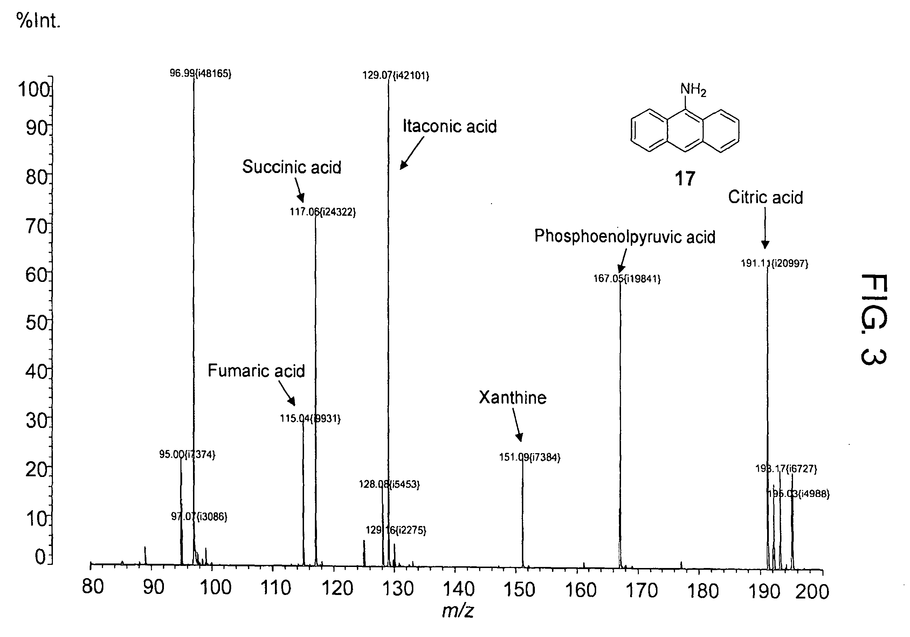

Fig. 3 shows a MALDI mass spectrometry spectrum of a mixture of 34 anionic biological components such as carboxylic acids (see Table 2 shown above about the composition thereof). Observations are made of respective peaks of fumaric acid, succinic acid, itaconic acid, xanthine, phosphoenolpyruvic acid, and citric acid.

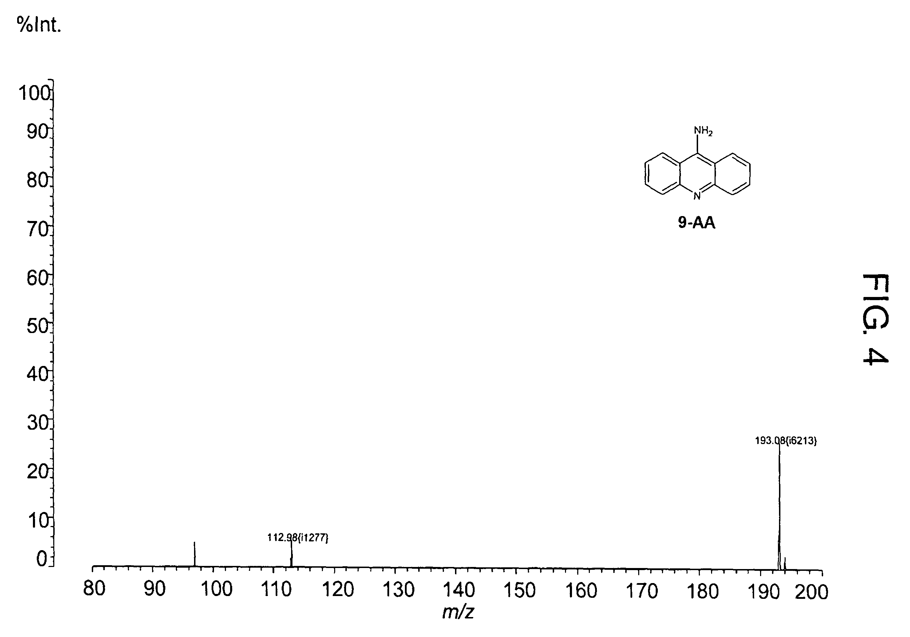

By contrast, Fig. 4 shows a result obtained by using 9-aminoacridine (abbreviated to 9-AA hereinafter), which is a typical matrix of conventional negative-ion-mode measurement, to make MALDI mass spectrometry of the same mixture. However, mass peaks are hardly observed. It is evident from this matter that 9-aminoanthracene is more useful than 9AA for detecting low-molecular-weight biological components in a negative ion mode.

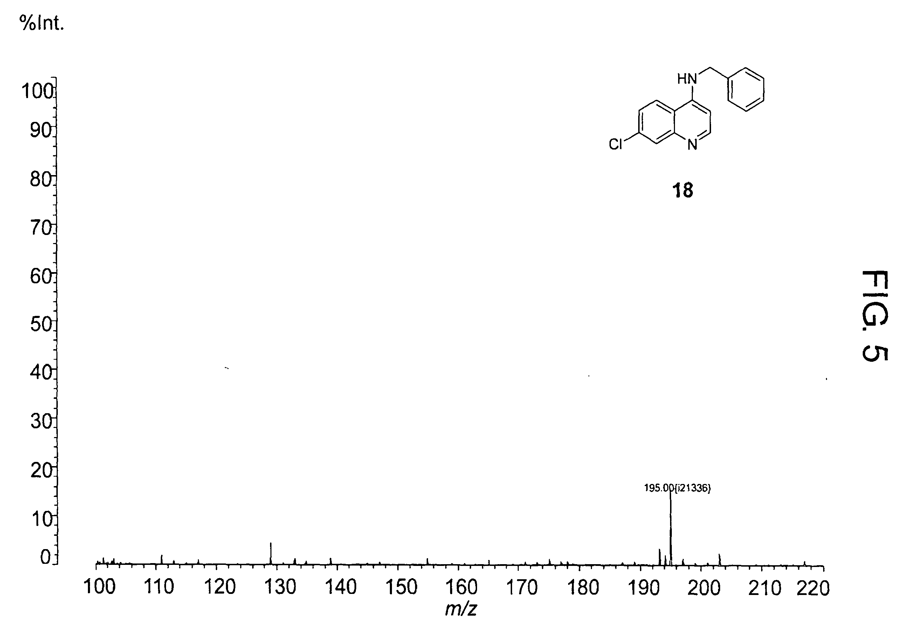

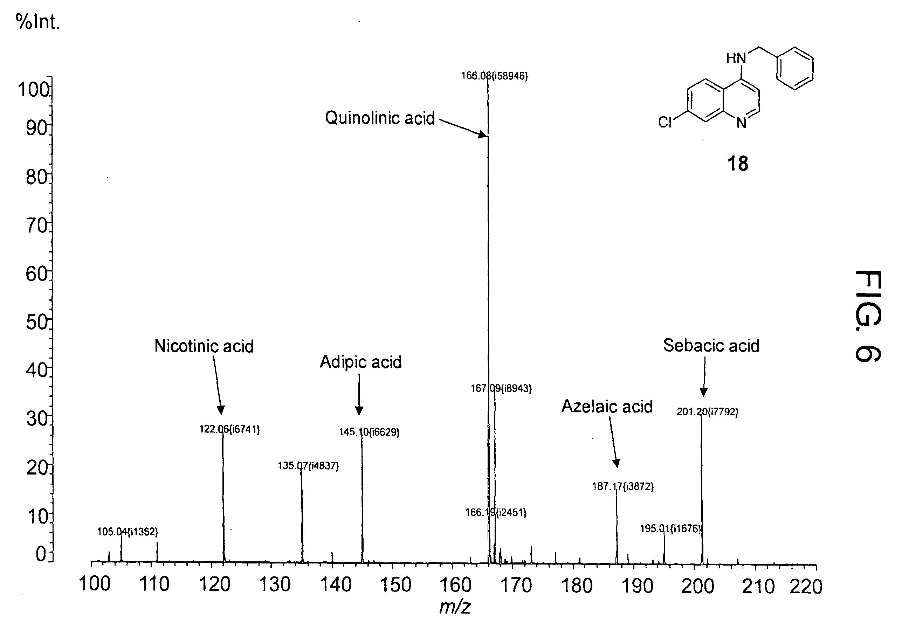

Fig. 5 is a chart showing a result of a blank measurement in the case of using 7-chloro-4-(N-benzylamino)quinoline (18) as a matrix according to the present invention. Remarkable peaks are not observed between m/z values of 100 and 220. Fig. 6 shows a spectrum obtained by using 7-chloro-4-(N-benzylamino)quinoline (18) as a matrix according to the present invention to make MALDI mass spectrometry of a mixture of approximately 30 anionic biological components (see Table 2 shown below about the composition thereof) in a negative ion mode. Observations are made of respective remarkable peaks of nicotinic acid, adipic acid, quinolinic acid, azelaic acid, and sebacic acid.

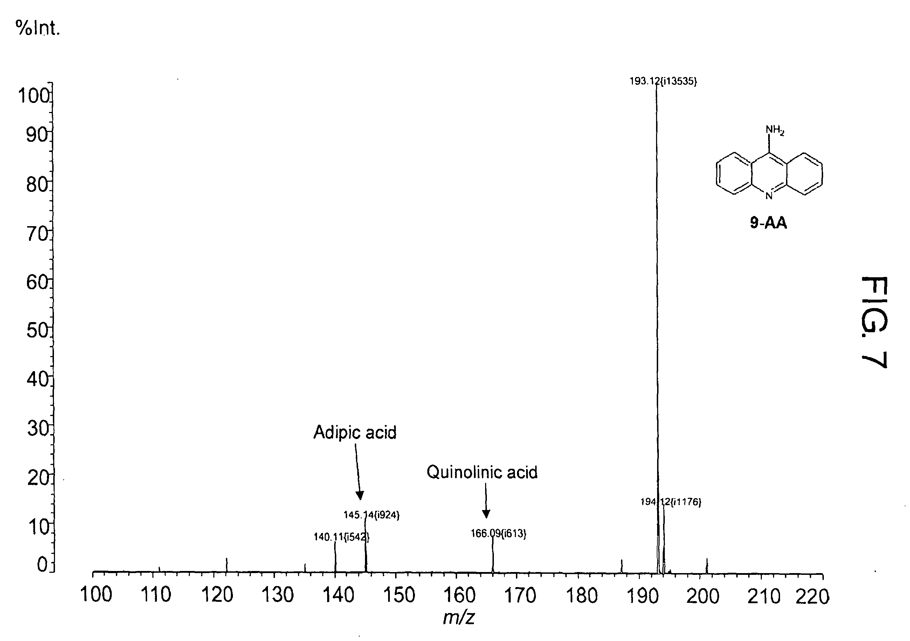

By contrast, Fig. 7 is a mass spectrum showing a result obtained by using 9-aminoacridine (9-AA) as a matrix to make MALDI mass spectrometry of the biological component mixture having the composition of Table 3 shown above in a negative ion mode. Observations are made of only weak peaks of adipic acid and quinolinic acid. It is clearly understood that 7-chloro-4-(N-benzylamino)quinoline (18) is more useful than 9AA as a matrix.

About the six anionic compound mixtures in Tables 1 to 6 shown above, various matrices were each used to make MALDI mass spectrometry in a negative ion mode. The results were compared with a result of a case where 9-AA was used as a matrix to make the same measurement, so that the order of some compounds was prepared in such a manner that about the compounds, the respective peak strength ratios between the case of using each of the matrices and that of using 9-AA were successively lined up from the largest value toward the smallest value. The orders obtained from the measurement results about the mixtures shown in Tables 1 and 2 shown above are shown in Tables 7 and 8, respectively. Each number described in each of the tables represents a matrix compound used for the measurement, and refers to the number of one of the matrix compounds in the present specification.

**[Table 7]**

| Compound | m/z | 1 | 2 | 3 | 4 | 5 |
|---|---|---|---|---|---|---|
| Serine | 104.04 | 17 | | | | |
| N-acetylglycine | 116.04 | 35 | 17 | 36 | 30 | 5 |
| Threonine | 118.05 | 17 | 35 | 18 | 24 | |
| Glutaric acid (pentanedicarboxylic acid) | 131.03 | 35 | 18 | 32 | 36 | 17 |
| Asparagine | 131.05 | 35 | 18 | 32 | 36 | 17 |
| Ornithine | 131.08 | 35 | 18 | 32 | 36 | 17 |
| Aspartic acid | 132.03 | 21 | 17 | 32 | 18 | 24 |
| Urocanic acid | 137.04 | 17 | 18 | 5 | 24 | 21 |
| Valine | 148.04 | 30 | | | | |
| Histidine | 154.06 | 35 | 36 | 17 | 21 | |
| N-acetylcysteine | 162.02 | 5 | 21 | | | |
| 3,4-Dihydroxyphenylacetic acid | 167.04 | 18 | | | | |
| 4-Hydroxyphenylpyruvic acid | 179.03 | 18 | | | | |
| Tyrosine | 180.07 | 35 | | | | |
| 5-Hydroxyindoleacetic acid | 190.05 | 36 | | | | |
| Phenylacetylglycine | 192.07 | 18 | 35 | 24 | 32 | 36 |
| Tryptophan | 203.08 | 37 | 10 | | | |
| Xanthurenic acid | 204.03 | 36 | 17 | 5 | 24 | 10 |
| N-acetylphenylalanine | 206.08 | 35 | 36 | 24 | 32 | 18 |
| N-acetyltyrosine | 250.11 | 35 | | | | |
| N-acetyl-aspartyl-glutamic acid (NAAG) | 303.08 | 10 | 5 | 36 | 21 | 13 |
| Fumaric acid | 115.00 | 29 | 17 | 18 | 5 | 21 |
| 5-Aminovaleric acid | 116.07 | 21 | 19 | | | |

**[Table 8]**

| Compound | m/z | 1 | 2 | 3 | 4 | 5 |
|---|---|---|---|---|---|---|
| Fumaric acid | 115.00 | 29 | 17 | 18 | 5 | 21 |
| 5-Aminovaleric acid | 116.07 | 21 | 19 | | | |
| Succinic acid | 117.02 | 29 | 17 | 5 | 18 | 30 |
| 5-Oxoproline | 128.04 | 17 | | | | |
| Itaconic acid (methylenesuccinic acid) | 129.02 | 29 | 17 | 5 | 32 | 24 |
| Agmatine | 129.11 | 29 | 17 | 5 | 32 | 24 |
| Xanthine | 151.03 | 29 | 24 | 34 | 5 | 30 |
| Thymine | 157.01 | 29 | | | | |
| Phosphoenolpyruvic acid (PEP) | 166.98 | 36 | 24 | 18 | 16 | 17 |
| Citric acid | 191.02 | 18 | 17 | 10 | 24 | 31 |
| Gluconic acid | 195.05 | 16 | 24 | 30 | | |
| Ribose 5-phosphoric acid | 229.01 | 36 | 31 | | | |
| Choline | 102.09 | 9AA | | | | |
| Uracil | 111.02 | 17 | 30 | | | |

In the case of using 9-AA, which is a matrix according to the prior art, many compounds cannot be detected. By contrast, it has been demonstrated that the matrix compounds created in the invention cause most of the anionic compounds to be efficiently ionized, so that these compounds can be detected in a wide range with a high sensitivity. From these results, it has been understood that the compounds 17, 18 and 21 according to the invention have a particularly high ionizing ability. It has been verified that, in particular, the compound 18 has a remarkable ionizing ability for many materials to be analyzed.

### Example (2) of spectrum analysis:

cis-Cinnamic acid, which is a substance acting on plants, and analogues thereof (see Table 9) were subjected to MALDI mass spectrometry in a negative ion mode to evaluate an effect of each of the matrix compounds 41 and 42 that was produced on the ability of ionizing each of the anionic compounds and on the peak strength thereof. Each of the carboxylic acids was mixed with the matrix at a ratio selected at will. Thereafter, the mixture was naturally dried on a stainless steel plate for MALDI. This sample was measured using a MALDI mass spectrometer (MALDI-TOF-MS: AXIMA, Performance, manufactured by Shimadzu Corp.).

**[Table 9]**

| Compound | m/z |
|---|---|
| cis-Cinnamic acid | 148.0524 |
| cis-Methoxymethyl cinnamate | 192.0786 |
| 3-Iodo-cis-cinnamic acid | 273.9491 |
| 3-Trifluoromethyl-cis-cinnamic acid | 216.0398 |
| Z-tetralin-1-ylidene acetic acid | 188.0837 |
| 3,4-Dihydronaphthalene-1-acetic acid | 188.0837 |
| Ethyl 3,4-Dihydronaphthalene-1-acetate | 216.115 |
| (Z)-3-(benzofuran-5-yl)propenoic acid | 190.063 |
| (Z)-3-(2,3-dihydrobenzofuran-6-yl)propenoic acid | 188.0473 |

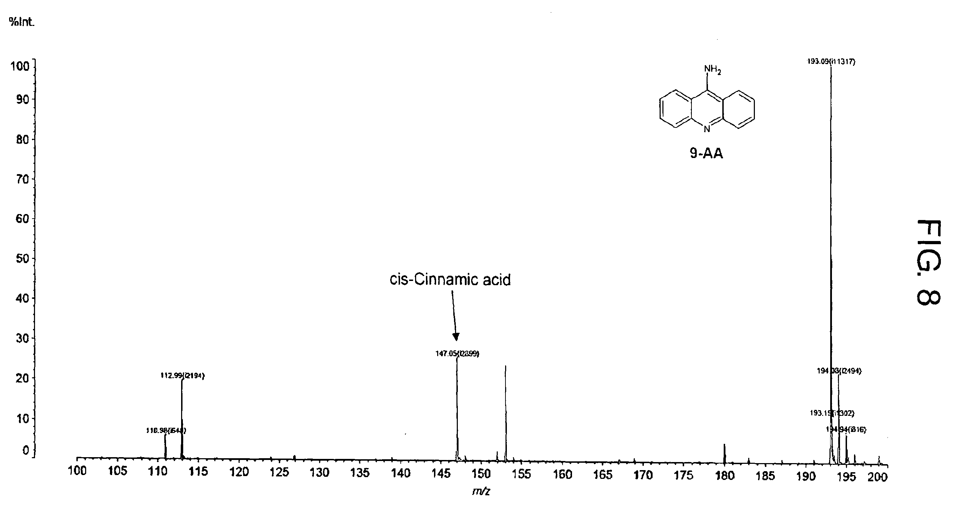

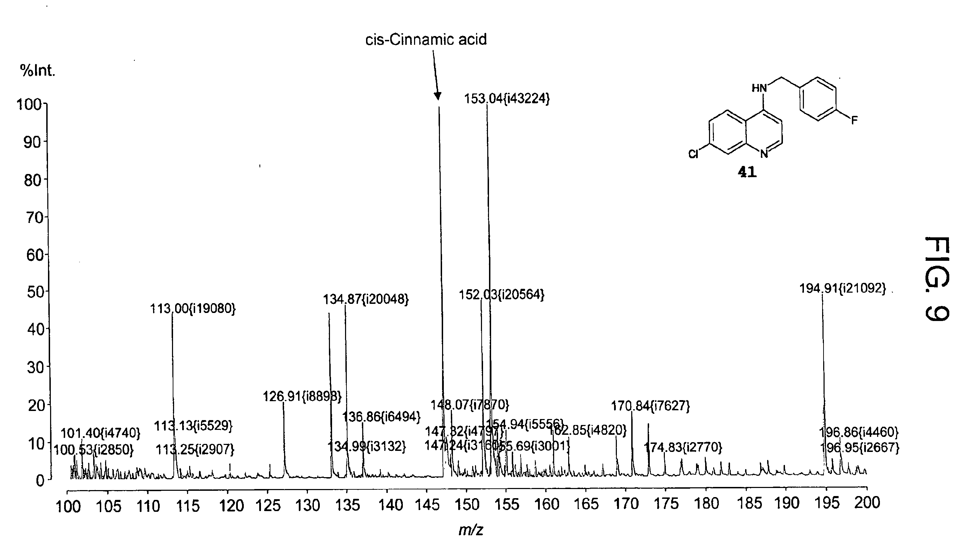

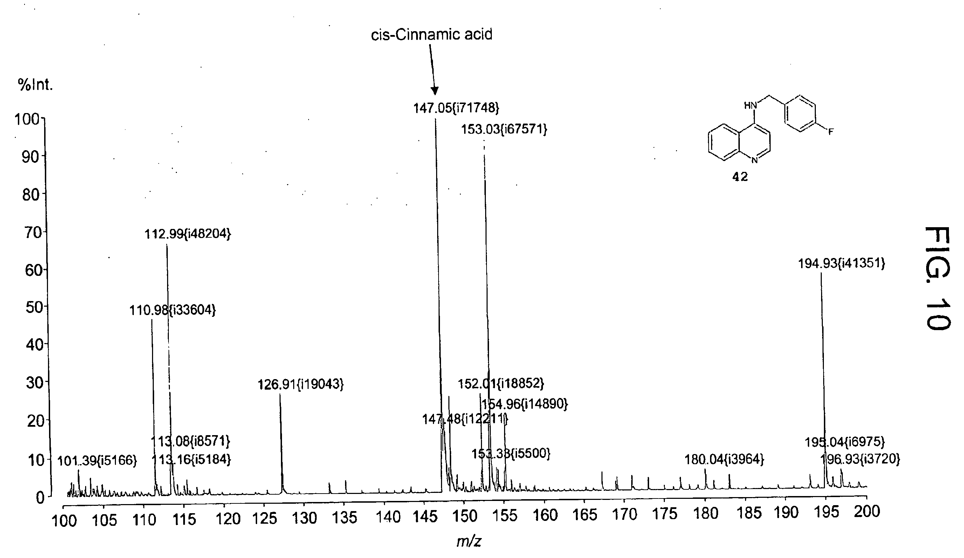

Figs. 8 to 10 each show a measurement result of cis-cinnamic acid. In the case of using 9-AA, which has been hitherto used as a matrix in negative-ion-mode measurement, a sufficient peak strength (m/z = 147.05: [M-1]⁻) is not obtained as illustrated in Fig. 8. By contrast, in the case of using each of the compounds41 and 42 as a matrix, the compound has a higher ionizing ability as illustrated in Figs. 9 and 10. Thus, it is understood that these matrices make it possible to make MALDI mass spectrometry with a high sensitivity. About the other carboxylic acids shown in Table 9 also, in the same manner as in the case of cis-cinnamic acid, it has become possible to attain a high-sensitivity MALDI mass spectrometry in a negative ion mode, which has not been easily attained using 9-AA as a matrix.

In MALDI mass spectrometry measurement of low-molecular-weight biological components, 2,5-dihydroxybenzoic acid (DHB) is frequently used as a matrix. However, it is not said that the compound is high in ionizing ability. Thus, many molecules are not detected therewith. In recent years, it has been shown that when 9-aminoacridine (9-AA) is used as a matrix in negative-ion-mode measurement, various low-molecular-weight biological components can be analyzed with a relatively high sensitivity (see, for example, Non-Patent Document 1). However, according to 9-AA, many compounds still cannot be measured. Thus, it has been desired to develop a higher-performance matrix for metabolome analysis, for which a rapid and high-sensitivity analysis is required. According to the present invention, the detection of low-molecular-weight compounds originating from living bodies, which have not been easily detected in MALDI mass spectrometry, has been successfully achieved by synthesizing the aminoquinoline derivatives as specified in claim 1, which show a higher ionizing ability and sensitivity than 9-aminoacridine. Moreover, the selection of a matrix suitable for a biological component as a target makes it possible to avoid the disturbance of peak detection that is based on peaks of ions of the matrix itself. The present invention is particularly useful for the detection or bio-imaging of a specific minor biological component.

Results obtained so far have suggested that an amino group on a quinoline is desired for a requirement of a matrix. By changing a substituent on the aromatic ring or a substituent on the amino group, the ionizing ability or the sensitivity can be adjusted. The amino group is a secondary amino group. The substituent on the amino group is desirably an aryl or benzyl group. A salt (such as hydrochloride) of such an amine is also usable. Any one of these compounds is commercially available, or can easily be synthesized through several steps from a commercially available material.

## Claims

1. A matrix for matrix-assisted laser desorption/ionization (MALDI) mass spectrometry, comprising:
a compound represented by the following 18 to 21, 25, 26, 41 or 42, or the salts thereof:

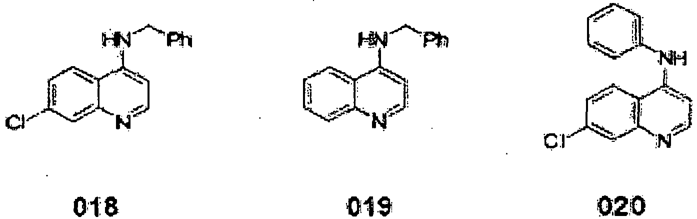

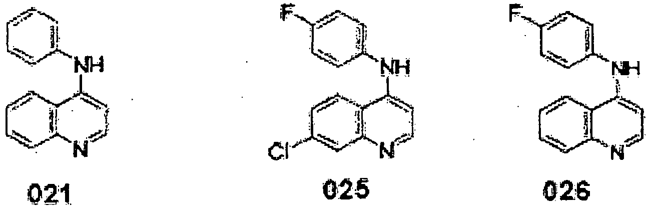

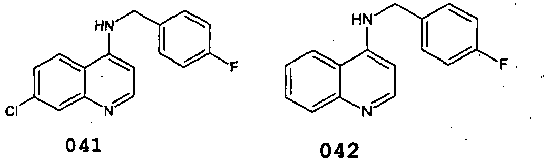

2. The matrix for matrix-assisted laser desorption/ionization (MALDI) mass spectrometry according to claim 1, comprising:
the compound represented by the following 18, or the salts thereof:

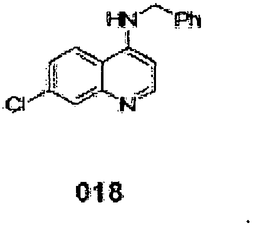

3. The matrix for matrix-assisted laser desorption/ionization (MALDI) mass spectrometry according to claim 1, comprising:
the compound represented by the following 41 or 42, or the salts thereof:

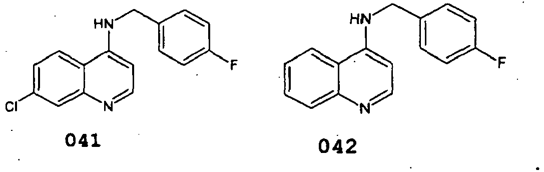

4. A MALDI mass spectrometry method of making MALDI mass spectrometry in a negative ion mode using the matrix according to any one of clams 1, 2 and 3.

5. The MALDI mass spectrometry method according to claim 4, wherein a material to be analyzed is an organic compound having a molecular weight of 1000 or less.

## Patentansprüche

1. Matrix für Massenspektrometrie mit Matrix-unterstützter Laser-Desorption/lonisation (MALDI), umfassend:
eine durch die folgenden 18 bis 21, 25, 26, 41 oder 42 dargestellte Verbindung oder deren Salze:

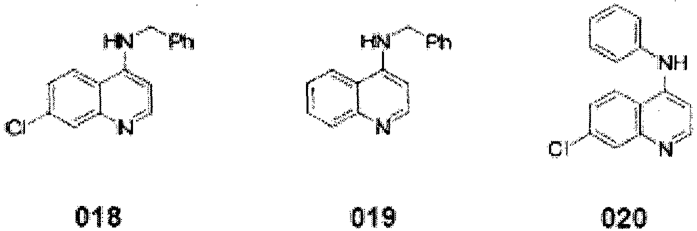

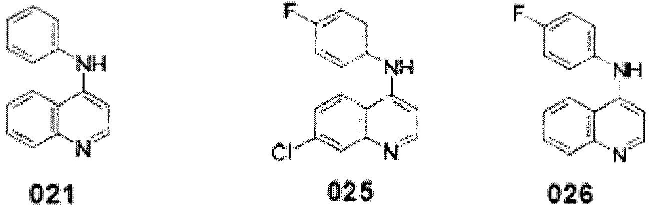

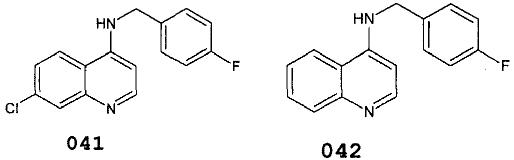

2. Matrix für Massenspektrometrie mit Matrix-unterstützter Laser-Desorption/Ionisation (MALDI) nach Anspruch 1, umfassend:
die durch die folgende 18 dargestellte Verbindung oder deren Salze:

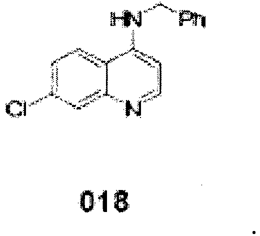

3. Matrix für Massenspektrometrie mit Matrix-unterstützter Laser-Desorption/Ionisation (MALDI) nach Anspruch 1, umfassend:
die durch die folgenden 41 oder 42 dargestellte Verbindung oder deren Salze:

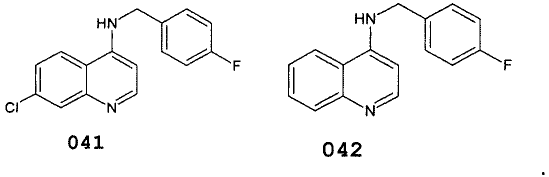

4. MALDI-Massenspektrometrieverfahren zum Durchführen einer MALDI- Massenspektrometrie im Modus mit negativen Ionen unter Verwendung der Matrix gemäß einem der Ansprüche 1, 2 und 3.

5. MALDI- Massenspektrometrieverfahren nach Anspruch 4, wobei ein zu analysierendes Material eine organische Verbindung mit einem Molekulargewicht von 1000 oder weniger ist.

## Revendications

1. Matrice pour spectrométrie de masse à désorption/ionisation par laser assistée par matrice (MALDI), comprenant :
un composé représenté par un des composés 18 à 21, 25, 26, 41 ou 42 suivants, ou leurs sels :

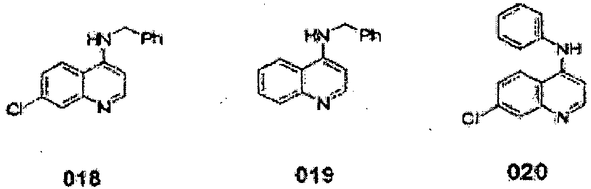

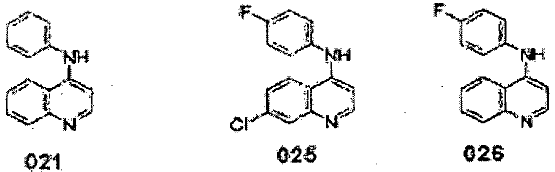

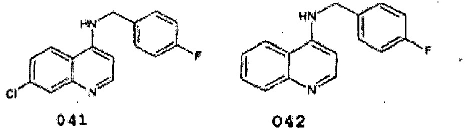

2. Matrice pour spectrométrie de masse à désorption/ionisation par laser assistée par matrice (MALDI) selon la revendication 1, comprenant :
le composé représenté par le composé 18 suivant, ou ses sels :

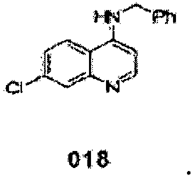

3. Matrice pour spectrométrie de masse à désorption/ionisation par laser assistée par matrice (MALDI) selon la revendication 1, comprenant :
le composé représenté par le composé 41 ou 42 suivant, ou leurs sels :

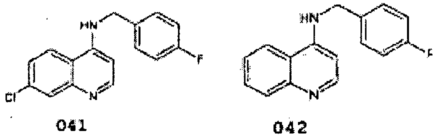

4. Procédé de spectrométrie de masse MALDI comprenant la préparation d'une spectrométrie de masse MALDI dans un mode à ion négatif en utilisant la matrice selon l'une quelconque des revendications 1, 2 et 3.

5. Procédé de spectrométrie de masse MALDI selon la revendication 4, dans lequel une substance à analyser est un composé organique ayant un poids moléculaire de 1000 ou moins.
